# EUROPEAN PATENT SPECIFICATION

(11) **EP 4 032 553 B1**
(45) Date of publication and mention of the grant of the patent: **07.08.2024**
(21) Application number: 21802581.5
(22) Date of filing: 23.08.2021
(51) Int. Cl.: A61L 2/10, H05B 47/115, H05B 47/13, H05B 47/16, H05B 47/165, H05B 47/17, A61L 9/20, A61L 2/24

(54) **MICROBE AND VIRUS INACTIVATION DEVICE**
MIKROBE UND VIRUSINAKTIVIERUNGSVORRICHTUNG
DISPOSITIF D'INACTIVATION DE MICROBES ET DE VIRUS

(30) Priority: 25.11.2020 JP 2020195381; 25.11.2020 JP 2020195386
(43) Date of publication of application: 27.07.2022
(73) Proprietor: Ushio Denki Kabushiki Kaisha, Tokyo 100-8150 (JP)
(72) Inventor: OKUMURA, Yoshihiko, Tokyo 1008150 (JP)
(74) Representative: Maiwald GmbH
(86) International application number: PCT/JP2021/030779
(87) International publication number: WO 2022/113442

(56) References cited:
- WO-A1-2018/131582
- WO-A1-2019/164810
- WO-A1-2019/186880
- WO-A1-2022/030342
- JP-A- 2018 517 488
- US-A1- 2018 193 504
- US-A1- 2018 373 157

## Description

### TECHNICAL FIELD

The present invention relates to an inactivating apparatus for bacteria or viruses.

### BACKGROUND ART

Bacteria (including fungi, etc.) and viruses that exist in spaces or on object surfaces may cause infectious diseases in humans or non-human animals, and there is a concern that the spread of such infectious diseases may threaten people's lives. Particularly, infectious diseases are likely to spread in facilities where people frequently gather, such as medical facilities, schools, and government offices, as well as in vehicles such as cars, trains, buses, airplanes, and ships, and therefore effective means of inactivating bacteria or viruses are needed.

Conventionally, irradiation with ultraviolet light has been known as a method to inactivate bacteria and viruses (hereinafter may collectively be referred to as "germs"). DNA has an absorption maximum around a wavelength of 260 nm. A low-pressure mercury lamp has a strong emission spectrum around a wavelength of 254 nm. Thus, a technique for sterilizing with a low-pressure mercury lamp has been widely used.

However, exposure of a human body to ultraviolet light in such a wavelength band is known to pose a risk of affecting the human body. The skin is divided into three parts from superficial to deep: the epidermis, the dermis, and the hypodermis deeper than the dermis, and the epidermis is further divided into four layers from superficial to deep: the stratum corneum, the stratum granulosum, the stratum spinosum, and the stratum basale. When a human body is exposed to ultraviolet light with a wavelength of 254 nm, the ultraviolet light penetrates the stratum corneum, reaches the stratum granulosum or the stratum spinosum, and in some cases the stratum basale, and is absorbed by DNA of the cells within these layers. This results in a risk of skin cancer. Therefore, it is difficult to actively use ultraviolet light in such a wavelength band in places where people can be present.

Patent Document 1 below describes that ultraviolet light with a wavelength of 240 nm or more (UVC light) is harmful to a human body and that the degree of influence of ultraviolet light with a wavelength of less than 240 nm on a human body is suppressed compared to ultraviolet light with a wavelength of 240 nm or more. Specifically, the results of irradiation experiments at wavelengths of 207 nm and 222 nm are described in Patent Document 1.

Additionally, Patent Document 2 below discloses the contents of irradiating ultraviolet light (UVC light) to the toilet, which is a space to be decontaminated, after detecting the absence of a person in the toilet. US 2018/193504 discloses a UV sanitising device, wherein the controller controls the light source to emit long interval, low intensity light when people are around, and high intensity short interval light when people are not proximate to the surface to be sterilised.
US 2018/373157 discloses a far-UVC germicidal device, wherein the controller controls the light source to activate in absence of a human being. During an activation cycle, the light source is configured to emit light in a range between a minimum intensity and a maximum intensity, in response to the value of irradiance on the surface to be disinfected, measured by a sensor, so to compensate for losses of antimicrobial efficacy due for example, to the aging of the light source.

### PRIOR ART DOCUMENT

### PATENT DOCUMENTS

Patent Document 1: Japanese Patent No. 6025756
Patent Document 2: JP-T-201 7-528258

### SUMMARY OF THE INVENTION

### PROBLEMS TO BE SOLVED BY THE INVENTION

The purpose of the present invention is to provide an inactivating apparatus for bacteria or viruses which is capable of efficiently inactivating bacteria or viruses in a space where a human being may be present while taking impacts on the human body into consideration.

### MEANS FOR SOLVING THE PROBLEMS

An inactivating apparatus for bacteria or viruses according to the present invention includes a light source that emits ultraviolet light with an optical output in a specific wavelength band belonging to a range of 190 nm to 235 nm; a controller for lighting control of the light source; and a detector that detects whether or not a human being is present in a detection target area, which is a part of an irradiation area of the ultraviolet light or an area adjacent to the irradiation area, in which the controller controls the light source to increase an integrated irradiation dose of the ultraviolet light per unit time when the detector detects the absence of a human being.

In this specification, "inactivation" is a concept that encompasses killing bacteria or viruses, or causing them to lose their infectivity or toxicity, and "bacteria" refers to microorganisms including bacteria and fungi (molds). In the following, "bacteria or viruses" may be collectively referred to as "germs".

As of the filing date of this application, the threshold limit value (TLV) of an ultraviolet light irradiation dose per day (8 hours) for a human body is specified depending on a wavelength by ACGIH (American Conference of Governmental Industrial Hygienists) or JIS Z 8812 (Measuring methods of eye-hazardous ultraviolet radiation). In other words, when ultraviolet light is used in an environment where a human being is present, it is recommended that the irradiance and irradiation time of ultraviolet light be determined so that the integrated irradiation dose of the ultraviolet light within a predetermined time is equal to or less than the standard value of TLV.

On the other hand, in a time zone during which a human being is absent in an area irradiated with ultraviolet light (irradiation area), there is no need to consider the standard value of TLV, even if the irradiance of ultraviolet light is increased or the irradiation time is extended. The effect of inactivating germs in the space enhances as the integrated irradiation dose of ultraviolet light within a certain base time increases.

According to Patent Document 1 described above, the degree of impact on a human body is suppressed by using ultraviolet light in a wavelength band shorter than 240 nm. The inactivating apparatus according to the present invention is designed to inactivate bacteria or viruses while suppressing their impact on a human body by using ultraviolet light having an optical output in a specific wavelength band belonging to a range of 190 nm to 235 nm.

Patent Document 2 only mentions the use of UVC light, and the details of its wavelength are unknown. However, given that, as described above, a wavelength band around 254 nm is most generally used heretofore to inactivate germs, that document only mentions UVC light and does not specifically describe the wavelength, and that the control to irradiate ultraviolet light is executed after confirming the absence of a human being, etc., it is natural to assume that the wavelength of ultraviolet light used in Patent Document 1 is around 254 nm.

From the viewpoint of achieving the inactivation effect while ensuring safety to a human body, the light source equipped with the inactivating apparatus is more preferably configured to emit ultraviolet light in a wavelength band of 200 nm to 230 nm. It is believed that irradiation with ultraviolet light in such a wavelength range has little impact on the human body even when the irradiation time is relatively long.

The inactivating apparatus according to the present invention emits ultraviolet light having an optical output in a wavelength range of 190 nm to 235 nm, and therefore the acceptable dose of ultraviolet light for a human body is much higher as compared to ultraviolet light having an optical output in a wavelength range of 240 nm or more. As a result, by controlling the irradiation dose within the range not exceeding TLV, the inactivating effect can be enhanced while suppressing the impact on a human body.

Furthermore, according to the above structure, the integrated irradiation dose of ultraviolet light is controlled to increase in a time zone during which a human being is absent, which makes it possible to enhance the inactivation effect of germs while suppressing the impact on a human body.

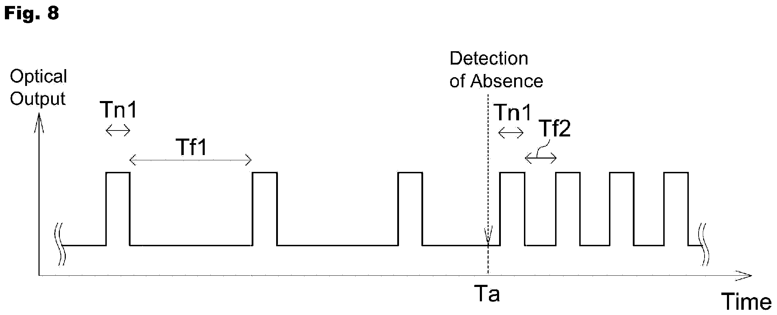

Note that the "integrated irradiation dose of ultraviolet light per unit time" in this specification means the following time. In the case where the light source is controlled by the controller to turn on periodically, and the lighting cycle is adjusted so that it differs between a time zone during which a human being is present and a time zone during which a human being is absent, the "integrated irradiation dose of ultraviolet light per unit time" refers to a value obtained by dividing an integrated irradiation dose in each lighting cycle (one cycle) by the lighting cycle. Taking Fig. 8, which will be described later in "MODE FOR CARRYING OUT THE INVENTION", as an example, one cycle in a time zone during which a human being is present is defined as the total of time Tn1 and time Tf1 (here conveniently referred to as a cycle τa). On the other hand, one cycle in a time zone during which a human being is absent is defined as the total of time Tn1 and time Tf2 ((here conveniently referred to as a cycle rb). In this case, in the time zone during which a human being is present, a value obtained by dividing an integrated irradiation dose during the time of the cycle ra by the cycle ra is defined as an integrated irradiation dose per unit time. On the other hand, in the time zone during which a human being is absent, a value obtained by dividing an integrated irradiation dose during the time of the cycle τb by the cycle τb is defined as an integrated irradiation dose per unit time. That is, the "integrated irradiation dose of ultraviolet light per unit time" corresponds to the average of an integrated irradiation dose in each lighting cycle.

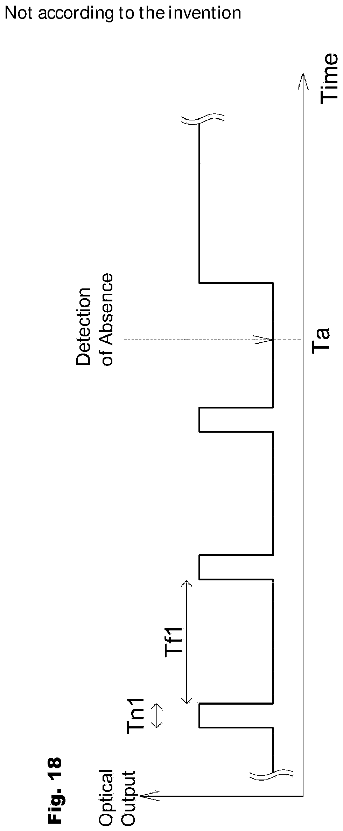

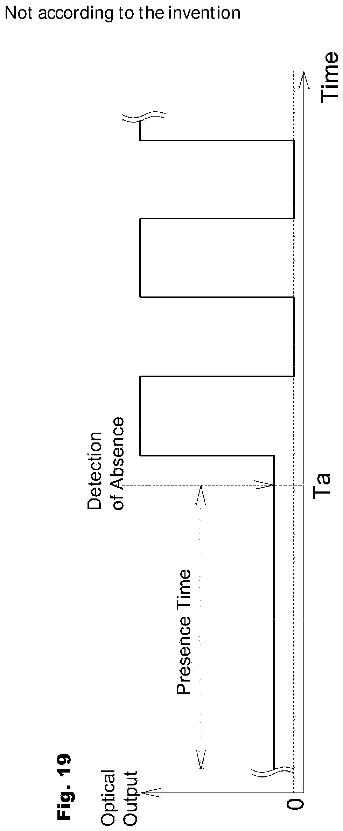

Not according to the invention:
In the case where the light source is controlled by the controller to turn on periodically only in a time zone during a human being is present, more specifically in a case shown in Fig. 18 described later in "MODE FOR CARRYING OUT THE INVENTION", a value obtained by dividing the integrated irradiation dose of ultraviolet light within a time corresponding to one cycle by the time corresponding to one cycle is set as the "integrated irradiation dose of ultraviolet light per unit time". On the other hand, also in the case where the light source is controlled by the controller to turn on periodically only in a time zone during which a human being is absent, more specifically, also in a case shown in Fig. 19 described later in "MODE FOR CARRYING OUT THE INVENTION", a value obtained by dividing the integrated irradiation dose of ultraviolet light within a time corresponding to one cycle by the time corresponding to one cycle is set as the "integrated irradiation dose of ultraviolet light per unit time".

Even if the light source is not periodically controlled to turn on by the controller, the "unit time" may be set in the same manner as described above if the light source is configured to allow periodic lighting control.

not according to the invention:
in the case where the light source is not periodically controlled to turn on by the controller and is configured to be unable to periodically controlled to turn on, in other words, in the case where the light source is subjected to only continuous lighting control when turned on, the "integrated irradiation dose of ultraviolet light per unit time" is set by dividing the integrated irradiation dose of ultraviolet light within an arbitrarily set time (e.g., 5 minutes, 10 minutes, etc.) by the set time (e.g., 5 minutes, 10 minutes, etc. in the above example). In this case, the "integrated irradiation dose of ultraviolet light per unit time" corresponds to the average value of an integrated irradiation dose during a continuous lighting operation.

The controller is configured to be capable of executing a specific operation mode in which a first control to turn on the light source at a relatively high emission intensity and a second control to turn on the light source at a relatively lower emission intensity than that during the execution of the first control or to turn off the light source are repeated, and
the controller continues to execute the specific operation mode with the integrated irradiation dose of the ultraviolet light per unit time increased when the detector detects the absence of a human being during the execution of the specific operation mode.

When ultraviolet light irradiation is continuously performed at a high intensity, heat generation in a power supply unit for supplying electric power to the light source becomes remarkable, and thus a large cooling system is required. Therefore, from the viewpoint of reducing the scale of the apparatus, the inactivating apparatus is preferably configured to be able to execute an operation mode (hereinafter referred to as a "specific operation mode") in which the irradiance of ultraviolet light is switched between high and low or ultraviolet light is switched between irradiation and non-irradiation.

Some germs can repair DNA damage when irradiated with light in the wavelength range of 300 nm to 500 nm after being inactivated by exposure to ultraviolet light with a wavelength of, for example, 254 nm. This is due to the function of photolyases (e.g., FAD (flavin adenine dinucleotide)) in the germs, and hereinafter this phenomenon is referred to as "photoreactivation of germs". Light in a wavelength range of 300 nm to 500 nm includes visible light of sunlight or white lighting, and it is known that the photoreactivation of germs is promoted in a bright environment. Due to the existence of such circumstances, when germs are inactivated by ultraviolet light irradiation in a lighted environment, it is often difficult to keep the germs in an inactive state. From such a viewpoint, when germs are inactivated using, for example, a conventional low-pressure mercury lamp, the lamp is required to be continuously turned on in principle.

However, the light source of the inactivating apparatus according to the present invention is configured to emit ultraviolet light having an optical output in a specific wavelength band belonging to a range of 190 nm to 235 nm. When an area where germs should be inactivated is irradiated with such ultraviolet light, "photoreactivation of germs" is less likely to occur even when the area is irradiated with the above-described visible light after ultraviolet light irradiation. In other words, such ultraviolet light has the effect of inhibiting "photoreactivation of germs". One of some conceivable reasons for this is that ultraviolet light in such a wavelength band acts on photolyase so that the photoreactivation function is inhibited. Therefore, even if the irradiance of ultraviolet light is temporarily reduced or ultraviolet light irradiation is intermittently performed during inactivation treatment, germs are less likely to progress in a time zone during which the irradiance is low or ultraviolet light is not irradiated. Accordingly, a high inactivating effect can be achieved while the scale of the apparatus is reduced.

According to the above structure, if the absence of a human being is detected during the period when the ultraviolet light is irradiated intermittently or the ultraviolet light is irradiated while switching between high and low intensity (i.e., during the execution of the specific operation mode), the specific operation mode is continuously executed with the integrated irradiation dose of the ultraviolet light increased. This achieves a compact and highly effective inactivating apparatus while taking impact on the human body into consideration.

From the viewpoint of further enhancing the effect of inhibiting photoreactivation function, the wavelength range in which ultraviolet light emitted from the light source has an optical output is more preferably in a range of 200 nm to 235 nm, even more preferably in a range of 200 nm to 230 nm, particularly preferably in a range of 215 nm to 230 nm.

The specific operation mode may be a mode in which an operation of executing the first control for a first predetermined time and then executing the second control for a second predetermined time is repeated, and
the controller may execute at least one of increasing the first predetermined time or decreasing the second predetermined time when the detector detects the absence of a human being.

According to the above structure, when the absence of a human being is detected, it is possible to intermittently perform ultraviolet light irradiation in a state where the integrated irradiation dose of ultraviolet light per unit time has been increased.

When the detector detects the absence of a human being, the controller may control to increase the optical output of the light source during the execution of the first control. More specifically, the "optical output" herein may be luminance.

This structure also makes it possible to increase the integrated irradiation dose of ultraviolet light per unit time when the absence of a human being is detected.

The ultraviolet light may have a peak wavelength in a range of 200 nm to 230 nm.

More specifically, the light source may be constituted from an excimer lamp filled with a light-emitting gas containing Kr and Cl or from an excimer lamp filled with a light-emitting gas containing Kr and Br. In the former case, the ultraviolet light has a peak wavelength of around 222 nm, and in the latter case, the ultraviolet light has a peak wavelength of around 207 nm. As another example, the light source may be constituted from a solid light source such as an LED or an LD.

By the way, in a time zone during which a human being is absent in the area where the ultraviolet light is irradiated (irradiation area), there is no need to consider the standard value of TLV even if the irradiance of ultraviolet light is increased or the irradiation time of ultraviolet light is prolonged. In addition, the higher the integrated irradiation dose of ultraviolet light, the more effective the inactivation of germs in the space.

That is, in order to enhance the effect of inactivating germs, there may be a procedure to continuously irradiate ultraviolet light at a high irradiance to increase the integrated irradiation dose after confirming the absence of a human being.

However, when ultraviolet light irradiation is continuously performed at a high irradiance for a long time, a power supply circuit including an inverter for supplying electric power to the light source generates heat which may require a large cooling system. If such a cooling system is provided, the inactivating apparatus itself may become larger and its installation place may be limited, which is not desirable. Furthermore, if the integrated irradiation dose per day becomes too high, the light source may reach the end of its life prematurely and the usable period may be shortened.

Additionally, as described above, some germs exhibit "photoreactivation" when irradiated with ultraviolet light in an environment where they are irradiated with visible light. Particularly, when inactivation treatment is performed in a space where a human being may come in and out, ultraviolet light irradiation is stopped in a time zone during which a human being is present, and thus there is a risk that germs may be reactivated by visible light irradiation in such a time zone. From such a viewpoint, in order to achieve a high inactivating effect with the conventional method, it is necessary to continuously perform ultraviolet light irradiation at a high irradiance in a time zone during which a human being is absent. This results in the need for a large cooling system to cool the power supply circuit as described above.

From such a viewpoint, it may also be considered to provide an inactivating apparatus that prevents an increase in the temperature thereof while achieving the effect of inactivating germs.

Therefore, the present invention is also directed to an inactivating apparatus for bacteria or viruses, including
a light source that emits ultraviolet light having an optical output in a specific wavelength band belonging to a range of 190 nm to 235 nm,
a controller for lighting control of the light source, and
a detector that detects whether or not a human being is present in a detection target area, which is a part of an irradiation area of the ultraviolet light or an area adjacent to the irradiation area, in which
the controller is configured to enable switching of a control content for the light source depending on a detection result obtained by the detector, and
when the detector detects the absence of a human being, the controller sets the control content to a first operation control mode, which repeats a first control and a second control, the first control of turning on the light source at a relatively high emission intensity, the second control of turning on the light source at a relatively lower emission intensity than that when executing the first control, or of turning off the light source.

Here, the "first operation control mode" may correspond to "the specific operation mode".

As described above, the light source equipped with the inactivating apparatus emits ultraviolet light having an optical output in a specific wavelength band belonging to a range of 190 nm to 235 nm, which exerts an inhibitory effect on the "photoreactivation of germs". Therefore, during the inactivation treatment, even if the ultraviolet light irradiation is intermittently performed due to, for example, the control of stopping the ultraviolet light irradiation in a time zone during which the presence of a human being is detected, in consideration of the effects on the human body, the growth of germs is unlikely to progress in a time zone during which the irradiance is low or the irradiation is not performed.

Compared with the conventional inactivating apparatus using ultraviolet light, this apparatus can achieve a higher inactivation effect even with a lower integrated irradiation dose of ultraviolet light in the same period.

As described above, in a time zone during which the detector detects the absence of a human being, this inactivating apparatus is set to the first operation control mode of repeating the first control and the second control, the first control of turning on the light source at a relatively high emission intensity, the second control of turning on the light source at a relatively lower emission intensity than when executing the first control or of turning off the light source. As a result, the amount of electric current flowing through the power supply circuit, such as an inverter, is suppressed during the execution of the second control, thus reducing the amount of heat generated by the power supply circuit. This makes it possible to achieve a high inactivating effect without providing a large cooling system.

On the other hand, there is a possibility that the inactivating apparatus is installed in a place where humans frequently come in and out. Therefore, if the light source is controlled so that the ultraviolet light irradiation is merely continuously performed at an extremely low irradiance in a time zone during which the absence of a human being is detected, then if the time when a human being is not present is short, the integrated irradiation dose of ultraviolet light per unit time may not be sufficient, and thus a high inactivation effect may not be obtained.

However, in the above structure, the first control, in which the light source is turned on at a relatively high emission intensity, and the second control, in which the light source is turned on at a relatively lower emission intensity than when executing the first control or in which the light is turned off, are repeated, thereby ensuring a high integrated irradiation dose within a short time when executing the first control, while suppressing the amount of heat generated in the power supply circuit when executing the second control.

Note that even when the light source is controlled to be turned off during the second control, "photoreactivation of germs" is less likely to occur when executing the second control, because irradiation with ultraviolet light having an optical output in a specific wavelength band belonging to a range of 190 nm to 235 nm is performed during the first control, as described above. Therefore, a high inactivating effect can be ensured.

not according to the invention: the controller may be configured to be able to set the control content to a second operation control mode in which the light source is turned off when the detector detects the presence of a human being and to shift the control content from the second operation control mode to the first operation control mode when the detector detects the absence of a human being.

When such an option is performed, the irradiation with ultraviolet light from the light source is stopped in a time zone during which a human being is present in the detection target area, thus minimizing the impact on the human body and psychological anxiety for a user. Furthermore, a high inactivating effect can be achieved while suppressing the temperature rise of the apparatus.

The first operation control mode may be a mode for repeating the operation of executing the first control of turning on the light source and then executing the second control of turning off the light source;
the controller may be configured to immediately shift the control content from the first operation control mode to the second operation control mode when the detector detects the presence of a human being during the execution of the first operation control mode; and
the controller may be configured to shift the control content from the second operation control mode to the first operation control mode to execute the first control after a transition waiting period set according to an elapsed time from the time when the control content was shifted from the first operation control mode to the second operation control mode when the detector detects the absence of a human being during the execution of the second operation control mode.

Note that "immediately" herein means after a lapse of, for example, 3 seconds or less, more preferably 1 second or less, particularly preferably 500 microseconds or less.

The timing at which the detector detects the absence of a human being is determined by the movement of the human being, and therefore cannot be controlled by the apparatus. That is, it can also be assumed that the human being stays in the detection target area for a very short time.

In such a situation, after the detector detects the presence of a human being and the ultraviolet light irradiation is stopped, the detector detects the absence of a human being after a lapse of a very short time. If the first operation control mode is executed immediately after the detector detects the absence of a human being so that the light source is turned on at a high emission intensity, the OFF time (light-off time) of the light source immediately before the start of turn-on will be very short. If such an operation is executed multiple times, the heat generation in the power supply circuit may increase.

In contrast, according to the above structure, when the detector detects the absence of a human being, the transition waiting period that elapses before the control content is shifted from the second operation control mode to the first operation control mode is set depending on the elapsed time from the time of the last detection of the presence of a human being. In other words, even when the absence of a human being is detected, the light source is turned on after remaining off for a while in the case where the elapsed time since the previous detection of the presence of a human being is short. This allows the heat generation of the power supply circuit to be suppressed regardless of the influence of an external factor that cannot be controlled by the inactivating apparatus, such as a human entering or leaving the detection target area.

Note that in the above structure, during the execution of the first operation control mode, that is, in a time zone during which a human being is absent, it is more preferable that the time when the light source is on (lit) is set to be shorter than the time when the light source is off (unlit) (a second off time). That is, during the execution of the first operation control mode, the ratio of the continuous lighting time of the light source (ON duty ratio) to the time required from once the light source is turned on to the next time is set to 50% or less. This suppresses the heat generation of the power supply circuit even during the execution of the first operation control mode.

The transition waiting period may be set so that a continuous light-off time from the immediately previous turn-off of the light source to the transition to the first operation control mode is equal to or longer than the continuous lighting time of the light source immediately before.

According to such a structure, even when a human being frequently enters and leaves the detection target area, the ON duty ratio is set to 50% or less, which enhances the effectiveness of suppressing the heat generation of the power supply circuit.

### EFFECT OF THE INVENTION

According to the present invention, it is possible to achieve an inactivating apparatus for bacteria or viruses which is capable of efficiently inactivating bacteria or viruses in a space where a human being may be present while taking impacts on the human body into consideration.

### BRIEF DESCRIPTION OF THE DRAWINGS

Fig. 1 is a schematic diagram showing an example of a situation in which an inactivating apparatus for bacteria or viruses according to the present invention is used.
Fig. 2 is a schematic diagram showing the structure of the inactivating apparatus.
Fig.3 is a functional block diagram for explaining the structure of the inactivating apparatus.
Fig. 4 is a schematic perspective view showing an example of the external appearance of a light source.
Fig. 5 is a schematic exploded perspective view showing a lamp house of the light source.
Fig. 6 is a schematic plan view showing the positional relationship between excimer lamps and electrodes.
Fig. 7 is a diagram showing an example of the spectrum of ultraviolet light emitted from the light source.
Fig. 8 is a timing chart schematically showing an example of the content of control executed by a controller on a light source in an inactivating apparatus according to a first embodiment.
Fig. 9 is a timing chart schematically showing another example of the content of control executed by the controller on the light source in the inactivating apparatus according to the first embodiment.
Fig. 10 is a timing chart schematically showing still another example of the content of control executed by the controller on the light source in the inactivating apparatus according to the first embodiment.
Fig. 11A is a graph showing the comparison of the effect of inactivating Staphylococcus aureus between continuous and intermittent irradiation of the bacterium with ultraviolet light with a wavelength of 254 nm.
Fig. 11B is a graph showing the comparison of the effect of inactivating Staphylococcus aureus between continuous and intermittent irradiation of the bacterium with ultraviolet light with a wavelength of 222 nm.
Fig. 12 is a schematic diagram showing another example of a situation in which the inactivating apparatus for bacteria or viruses according to the present invention is used.
Fig. 13 is a schematic diagram showing still another example of a situation in which the inactivating apparatus for bacteria or viruses according to the present invention is used.
Fig. 14 is a schematic diagram showingyet another example of a situation in which the inactivating apparatus for bacteria or viruses according to the present invention is used.
   Not according to the invention: Fig.15A to Fig. 19:
Fig. 15A is a timing chart schematically showing an example of the content of control executed by a controller on a light source in an inactivating apparatus according to a second embodiment.
Fig. 15B is another timing chart schematically showing an example of the content of control executed by the controller on the light source in the inactivating apparatus according to the second embodiment.
Fig. 16A is a timing chart schematically showing an example of the content of control executed by the controller on the light source in the inactivating apparatus according to the second embodiment when a transition waiting period is not provided.
Fig. 16B is a timing chart schematically showing an example of the content of control executed by the controller on the light source in the inactivating apparatus according to the second embodiment when a transition waiting period is provided.
Fig. 17A is another timing chart schematically showing an example of the content of control executed d by the controller on the light source in the inactivating apparatus according to the second embodiment when a transition waiting period is not provided.
Fig. 17B is a timing chart schematically showing an example of the content of control executed by the controller on the light source in the inactivating apparatus according to the second embodiment when a transition waiting period is provided.
Fig. 18 is a timing chart schematically showing still another example of the content of control performed by the controller on the light source.
Fig. 19 is a timing chart schematically showing yet another example of the content of control performed by the controller on the light source.

### MODE FOR CARRYING OUT THE INVENTION

Embodiments of an inactivating apparatus for bacteria or viruses according to the present invention will be described regarding the drawings. In the following description, the "inactivating apparatus for bacteria or viruses" may simply be referred to as the "inactivating apparatus".

### [First Embodiment]

A first embodiment of the inactivating apparatus will be described.

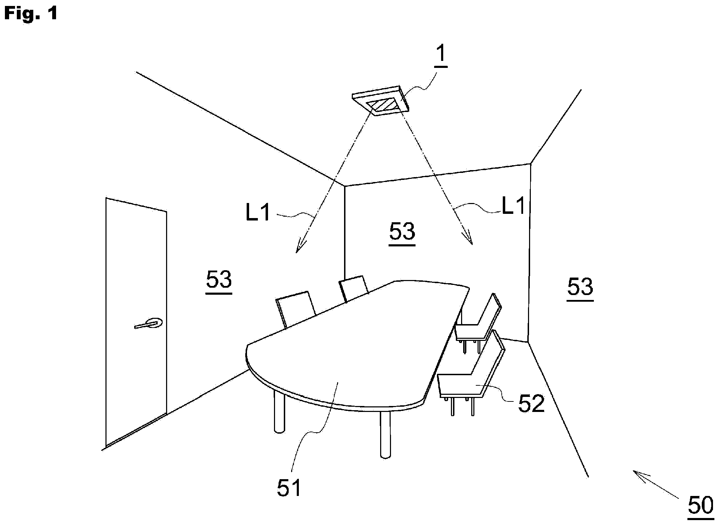

Fig. 1 is a schematic diagram showing an example of a situation in which the inactivating apparatus for bacteria or viruses according to the present invention is used. Fig. 1 shows, as an example, a situation in which an inactivating apparatus 1 is installed in a room 50 such as a meeting room. The inactivating apparatus 1 is installed to inactivate germs on a desk 51, chairs 52, and wallpaper 53 placed in the room 50, as well as inside the space of the room 50.

The inactivating apparatus 1 is configured to emit an ultraviolet light L1 that will be described later. When this ultraviolet light L1 is irradiated, inactivation treatment is performed on objects or spaces to be subjected to the inactivation of germs.

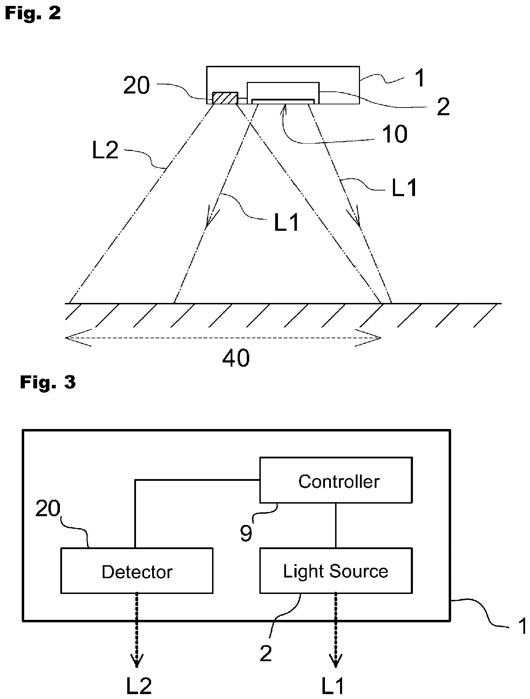

Fig. 2 is a schematic diagram showing the structure of the inactivating apparatus 1. Fig. 3 is a functional block diagram for explaining the structure of the inactivating apparatus 1.

The inactivating apparatus 1 includes a light source 2 that emits ultraviolet light L1, a detector 20 for detecting whether or not a human being is present in a detection target area 40, and a controller 9 that performs lighting control on the light source 2. The detection target area 40 is an area where the detector 20 detects whether or not a human being is present, and where at least a portion of the area can be irradiated with ultraviolet light L1. However, the detection target area 40 need not be completely coincident with the area to be irradiated with ultraviolet light L1. In other words, the detection target area 40 is a part of an irradiation area of the ultraviolet light L1 or an area adjacent to the irradiation area.

In this embodiment, the detector 20 is constituted from a human detection sensor using infrared light L2. The controller 9 changes the content of lighting control performed on the light source 2 when the detector 20 detects the absence of a human in the detection target area 40. The control content will be described later.

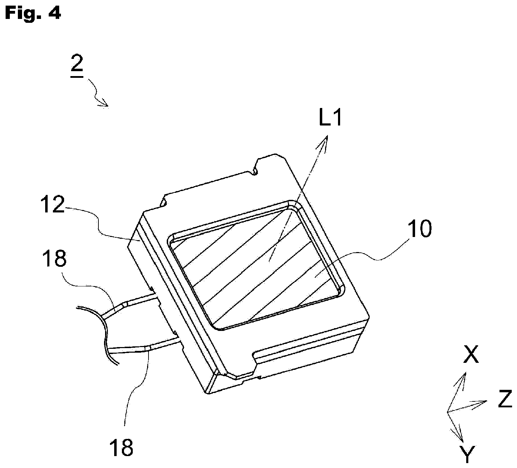

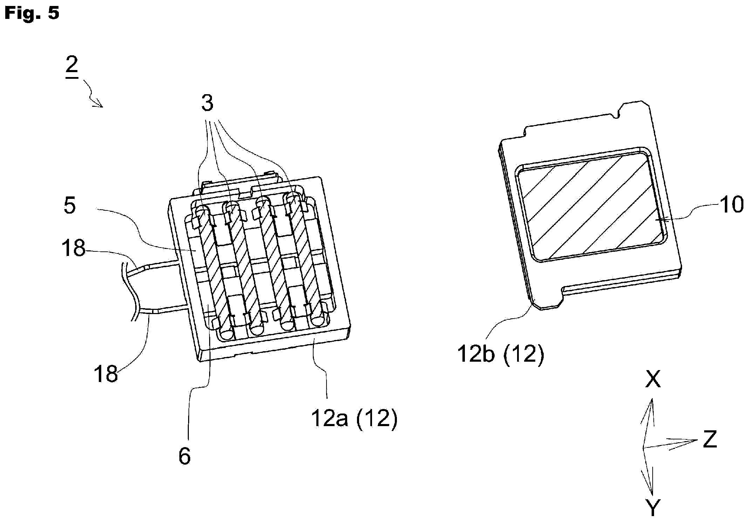

Fig. 4 is a schematic perspective view showing an example of the external appearance of the light source 2. Fig. 5 is an exploded perspective view of the light source 2 shown in Fig. 4, in which a lamp house 12 of the light source 2 is broken into a main body casing 12a and a lid 12b.

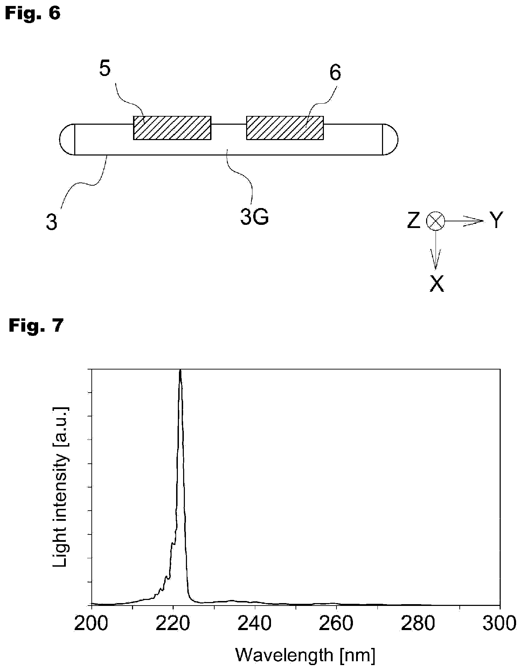

In the following explanation referring to Figs. 4 to 6, an X-Y-Z coordinate system is used, where an extraction direction of the ultraviolet light L1 is defined as an X direction, and a plane orthogonal to the X direction is defined as a YZ plane. More specifically, as will be described later referring to Figs. 5 and 6, a direction parallel to the tube axis of an excimer lamp 3 placed in the lamp house 12 is defined as a Y direction, and a direction orthogonal to the X and Y directions is defined as a Z direction.

As shown in Fig. 4 and Fig. 5, the light source 2 includes a lamp house 12 having a light extraction face 10 formed in one of the surfaces thereof. The lamp house 12 includes a main body casing 12a and a lid 12b. In the main body casing 12a, excimer lamps 3 and electrodes (5, 6) are housed. Fig. 5 shows, as an example, a case where four excimer lamps 3 are housed in the lamp house 12. The electrodes (5, 6) are electrically connected to power feeders 18 and constitute electrodes for feeding electric power to each of the excimer lamps 3. Fig. 6 is a schematic plan view showing the positional relationship between the excimer lamps 3 and the electrodes (5,6).

As shown in Figs. 4 to 6, the light source 2 in this embodiment has two electrodes (5, 6) arranged to contact the outer surface of the light-emitting tube of each of the excimer lamps 3. The electrodes (5, 6) are placed in positions apart from each other in the Y direction. The electrodes (5, 6) are made of an electrically conductive material and are preferably made of a material having the property of reflecting ultraviolet light L1 emitted from the excimer lamps 3. For example, the electrodes (5, 6) are both made of Al, an Al alloy, or stainless steel, etc. Both the electrodes (5, 6) are arranged to spread across all the excimer lamps 3 in the Z direction while contacting the outer surface of the light-emitting tube of each of the excimer lamps 3.

The excimer lamps 3 each have a light-emitting tube having a tube axis in the Y direction, and the outer surface of the light-emitting tube of each of the excimer lamps 3 is in contact with the electrodes (5, 6) in positions away from each other in the Y direction. The light-emitting tube of each of the excimer lamps 3 is filled with a light-emitting gas 3G. When a high-frequency AC voltage of, for example, about several kHz to 5 MHz is applied between each of the electrodes (5, 6) through the power feeders 18 (see Fig. 4) under control of the controller 9 (see Fig. 3), the voltage is applied to the light-emitting gas 3G through the light-emitting tube of the excimer lamps 3. At this time, discharge plasma is generated in a discharge space filled with the light-emitting gas 3G, and atoms of the light-emitting gas 3G are excited to an excimer state. When the atoms are returned to a ground state, excimer light is emitted.

The light-emitting gas 3G is made of a material that emits ultraviolet light L1 having an optical output in a specific wavelength band belonging to a range of 190 nm to 235 nm during emission of excimer light. For example, the light-emitting gas 3G contains KrCl, KrBr, or ArF.

For example, when the light-emitting gas 3G contains KrCl, ultraviolet light L1 having a main peak wavelength around 222 nm is emitted from the excimer lamps 3. When the light-emitting gas 3G contains KrBr, ultraviolet light L1 having a main peak wavelength around 207 nm is emitted from the excimer lamps 3. When the light-emitting gas 3G contains ArF, ultraviolet light L1 having a main peak wavelength around 193 nm is emitted from the excimer lamps 3. Note that the excimer lamp may be configured to emit ultraviolet light L1 with a wavelength longer than that of excimer light by applying a fluorescent material onto the tube walls of light-emitting tubes of the excimer lamps 3. Fig. 7 is a diagram showing an example of the spectrum of ultraviolet light L1 emitted from the excimer lamps 3 using the light-emitting gas 3G containing KrCI.

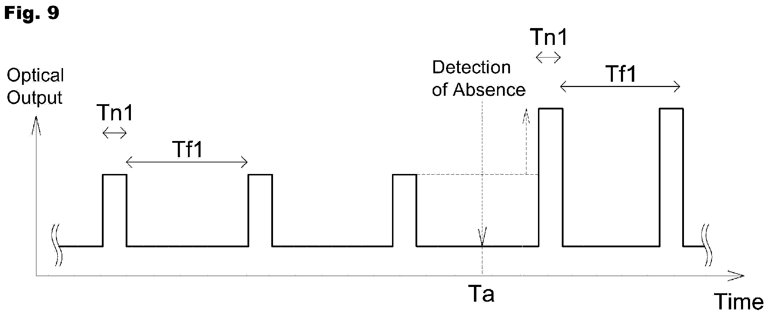

Fig. 8 is a timing chart schematically showing an example of the content of control performed by the controller 9 on the light source 2. More specifically, Fig. 8 schematically shows a change in the optical output of ultraviolet light L1 emitted from the light source 2 depending on the result of control performed by the controller 9. The method of graphical description in Fig. 8 is common to Fig. 9, Fig. 10, and Figs. 15A to Fig. 19 described later.

In this embodiment, the controller 9 controls the light source 2 so that a time when the light source 2 is energized (ON time) and a time when the light source 2 is not energized (OFF time) occur alternately. In other words, the controller 9 is configured to allow the light source 2 to execute an operation mode in which a turn-on operation and a turn-off operation are repeated (corresponding to a "specific operation mode"). In this case, the turn-on operation corresponds to "first control" and the turn-off operation corresponds to "second control". When explained using these terms, the specific operation mode is a mode to repeat an operation in which the first control is executed for a certain time and then the second control is executed for a certain time.

As an example, by energizing the light source 2 for a time Tn1 (corresponding to a "first predetermined time"), the light source 2 emits the ultraviolet light L1 for the time Tn1. Then, by stopping energization of the light source 2 is stopped for a time Tf1 (corresponding to a "second predetermined time"), the irradiation of the ultraviolet light L1 is stopped. For example, the time Tn1 is 15 seconds, and the time Tf1 is 250 seconds. These times can appropriately be changed by the controller 9.

In Fig. 8, a change in the optical output from the light source 2, that is, irradiation/non-irradiation with ultraviolet light L1 is illustrated in an extremely linear manner, but is just schematically shown for explanatory convenience. When analyzed along the time axis in detail, the optical output may smoothly decrease/increase. The speed (responsiveness) at which the optical output from the light source 2 fluctuates in response to a change in a control signal from the controller 9 depends on the structure of the light source 2. Fig. 8 merely schematically shows that the light source 2 is subjected to ON/OFF control based on the control signal from the controller 9, and therefore does not imply that the light source 2 has extremely high responsiveness. In other words, in the present invention, a speed of fluctuation of the optical output from the light source 2 based on the control signal from the controller 9 is not limited. This point is common to Fig. 9, Fig. 10, and Figs. 15A to Fig. 19 described later.

Assume that the detector 20 detects the absence of a human being in the detection target area 40 at a time Ta. When the controller 9 receives, from the detector 20, a signal indicating that the absence of a human has been detected, the controller changes the content of control performed on the light source 2 so that the integrated irradiation dose of ultraviolet light L1 in a predetermined unit of time increases.

More specifically, for example, the controller 9 controls to decrease the OFF time of the light source 2 after the time Ta (see Fig. 8). That is, the OFF time has been set to Tf1 before the time Ta, but is set to Tf2 shorter than Tf1 after the time Ta. As a result, the ratio of the continuous lighting time of the light source 2 (ON duty ratio) to the time required from once the light source 2 is turned on to the next time increases.

The detection of the absence of a human being in the detection target area 40 is not likely to cause an increase in the integrated irradiation dose of the ultraviolet light L1 irradiated to the human being, even when the integrated irradiation dose of ultraviolet light L1 per unit time is increased. Therefore, the effect of inactivating germs in the room 50 (see Fig. 1) can be enhanced without exceeding the standard value specified by ACGIH or JIS Z 8812 for the integrated irradiation dose of ultraviolet light L1 irradiated to a human being.

Although not shown in Fig. 8, the OFF time of the light source 2 is again set to Tf1 when the detector 20 detects the presence of a human being at a certain time Tb after that. This makes it possible to achieve the effect of inactivating germs while suppressing the integrated irradiation dose of ultraviolet light L1 to a human being within the standard value.

Particularly, the light source 2 is configured to emit ultraviolet light L1 having an optical output in a specific wavelength band belonging to a range of 190 nm to 235 nm, and in the case of ultraviolet light L1 in such a wavelength band, a higher inactivating effect can be achieved even when ultraviolet light irradiation is not continuously performed as compared to ultraviolet light with a wavelength of 254 nm emitted from a low-pressure mercury lamp or the like generally widely used for sterilization or the like. This point will be described later.

Fig. 9 is a timing chart showing another example of the content of control executed by the controller 9. As shown in Fig. 9, when the absence of a human being in the detection target area 40 is detected at a time Ta, a control to increase the optical output of the light source 2 (excimer lamps 3) may be performed. As a result, the luminance of the light source 2 increases, which makes it possible to increase the integrated irradiation dose of ultraviolet light L1 per unit time as compared to that before the time Ta. As a method to increase the optical output of the light source 2, for example, a method to increase the peak value of a pulse voltage applied to the light source 2 or a method to increase the frequency of the pulse voltage can be adopted.

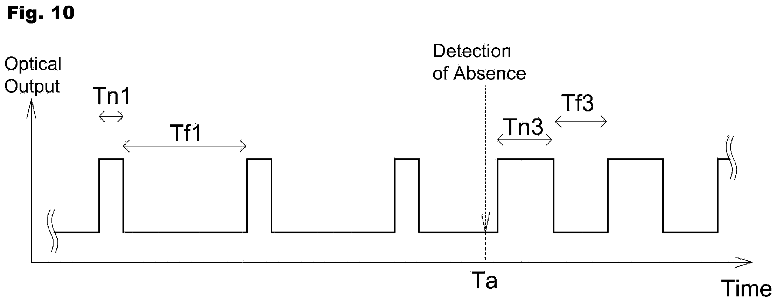

Fig. 10 is a timing chart showing still another example of the content of control executed by the controller 9. As shown in Fig. 10, when the absence of a human being in the detection target area 40 is detected at a time Ta, a control to increase the ON time of the light source 2 may be executed. That is, the ON time has been set to Tn1 before the time Ta, but is set to Tn3 longer than Tn1 after the time Ta. As a result, the ratio of the continuous lighting time of the light source 2 (ON duty ratio) to the time required from once the light source 2 is turned on to the next time increases.

In the example shown in Fig. 10, the OFF time is also set to Tf3 shorter than Tf1. However, a control may be executed with the OFF time remaining Tf1.

The control contents shown in Fig. 8 to Fig. 10 may appropriately be combined.

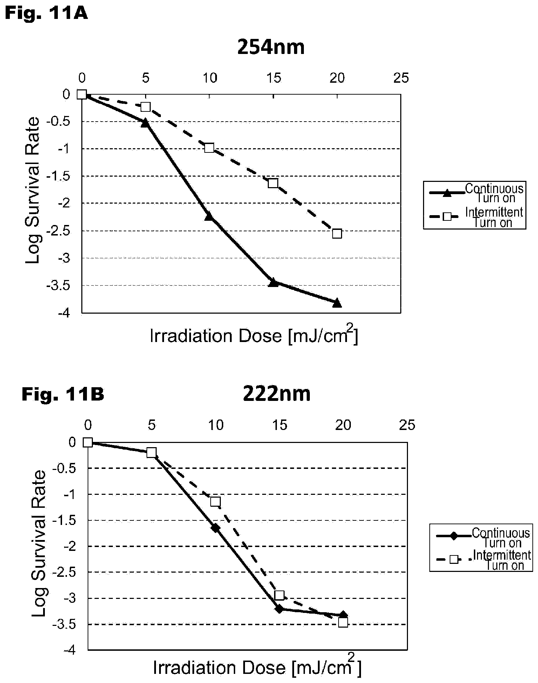

Figs. 11A and 11B are graphs for explaining that the effect of inactivating germs differs depending on the wavelength of ultraviolet light to be applied between continuous ultraviolet light irradiation and intermittent ultraviolet light irradiation. Both the graphs show the results of the following experiment.

One milliliter of a Staphylococcus aureus suspension having a concentration of about 10⁶ CFU/mL was placed in a 35 mmφ petri dish, and the petri dish was irradiated from above with ultraviolet light emitted from a low-pressure mercury lamp (Comparative Example) or ultraviolet light emitted from a KrCI excimer lamp (Example). Note that CFU stands for colony forming unit. Then, the suspension in the petri dish after irradiation was diluted with normal saline by a predetermined factor, and 0.1 mL of the suspension after dilution was inoculated on standard method agar. Then, the suspension was cultivated for 48 hours in a cultivation environment of a temperature of 37°C and a humidity of 70%, and the number of colonies was counted.

In both Comparative Example and Example, experiments were conducted both with continuous ultraviolet light irradiation and with intermittent ultraviolet light irradiation. The intermittent ultraviolet light irradiation was performed by repeating the control of irradiating for 50 seconds and then not irradiating for 59 minutes and 10 seconds. This experiment was conducted in an ordinary room without any blackout curtains.

Figs. 11A and 11B graphically show the results of the experiment, in which the horizontal axis corresponds to the irradiation dose of ultraviolet light and the vertical axis corresponds to the survival rate of Staphylococcus aureus. The vertical axis corresponds to the log value of the ratio of the number of colonies of Staphylococcus aureus after ultraviolet light irradiation to the number of colonies of Staphylococcus aureus before ultraviolet light irradiation.

As can be seen from Fig. 11A, in the case of ultraviolet light with a wavelength of 254 nm, the survival rate of Staphylococcus aureus is apparently higher when irradiated intermittently than when irradiated continuously. In contrast, in the case of ultraviolet light with a wavelength of 222 nm, even when irradiation is intermittently performed, the survival rate of Staphylococcus aureus is substantially the same as that when irradiation is continuously performed. Particularly, in this experiment, the irradiation mode was 50 seconds of irradiation followed by 59 minutes and 10 seconds of non-irradiation, and it was confirmed that the inactivation effect was almost equivalent to continuous ultraviolet light irradiation, even though the irradiation time within the unit time of 60 minutes was only 1.3% of the time.

The reason for this is presumably that DNA damage was repaired by photolyase in the bacteria by irradiation with illumination light or natural light in a time during which ultraviolet light irradiation is not performed after the bacteria were inactivated by irradiation with ultraviolet light with a wavelength of 254 nm. On the other hand, in the case of ultraviolet light with a wavelength of 222 nm, it is presumed that the ultraviolet light acts also on the photolyase, and therefore photoreactivation function is kept inhibited even in a time during which ultraviolet light irradiation is not performed. Such function is achieved as long as ultraviolet light L1 having an optical output in a specific wavelength band belonging to a range of 190 nm to 235 nm is used. Particularly, the function is high when the wavelength is 200 nm to 235 nm and is further conspicuously exhibited when the wavelength is 215 nm to 230 nm.

That is, according to the inactivating apparatus 1 of this embodiment, in a time zone during which the presence of a human being in the detection target area 40 is detected by the detector 20, the integrated irradiation dose per unit time is reduced by lowering the ON duty ratio of the light source 2 or lowering the optical output (luminance). As a result, the integrated irradiation dose of ultraviolet light applied to a human being is set without exceeding the standard value. Even in this time zone, the ultraviolet light L1 is irradiated intermittently, which ensures the effect of inactivating germs. On the other hand, in a time zone during which the absence of a human in the detection target area 40 is detected by the detector 20, the integrated irradiation dose per unit time is increased by increasing the ON duty ratio of the light source 2 or by increasing the optical output (luminance). As a result, the effect of inactivating germs is further enhanced without allowing the integrated irradiation dose of ultraviolet light applied to a human being to exceed the standard value.

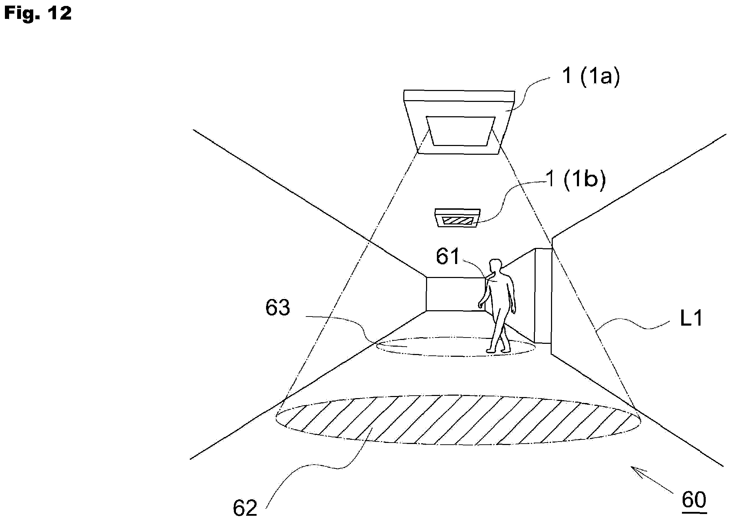

Figs. 12 to 14 are schematic diagrams each showing another example of a situation in which the inactivating apparatus 1 is used.

Fig. 12 shows a situation in which the inactivating apparatuses 1 are installed in an aisle 60 in a building or vehicle. The detector 20 of an inactivating apparatus 1a has not detected the presence of a human being 61, and therefore the ON duty ratio has been increased and an area 62 has been irradiated with ultraviolet light L1. On the other hand, the detector 20 of an inactivating apparatus 1b has detected the presence of the human being 61, and therefore the ON duty ratio has been reduced and an area 63 has not been irradiated with ultraviolet light L1 in a time zone shown by Fig. 12.

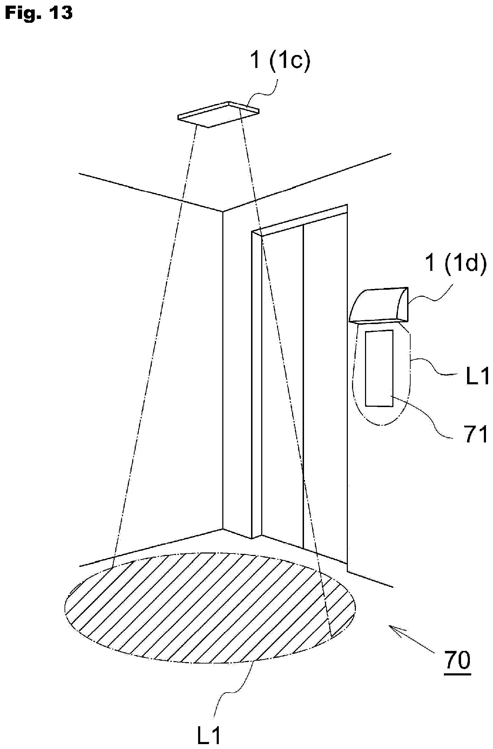

Fig. 13 shows a situation in which the inactivating apparatuses 1 are installed in a room 70. An inactivating apparatus 1c is installed for the purpose of performing inactivation of germs on, for example, the space or floor surface of the room 70. An inactivating apparatus 1d is installed for the purpose of performing inactivation of germs on an operating part 71 such as a remote controller for controlling air conditioning or lighting of the room 70. Also in this example shown in Fig. 13, when the inactivating apparatuses 1 (1c, 1d) detect the absence of a human being (not shown), the integrated irradiation dose of ultraviolet light L1 per unit time is increased, and therefore the effect of inactivating germs in the space or on the floor surface of the room 70 and germs on the operating part 71 is enhanced without allowing the integrated irradiation dose of ultraviolet light applied to a human being to exceed the standard value.

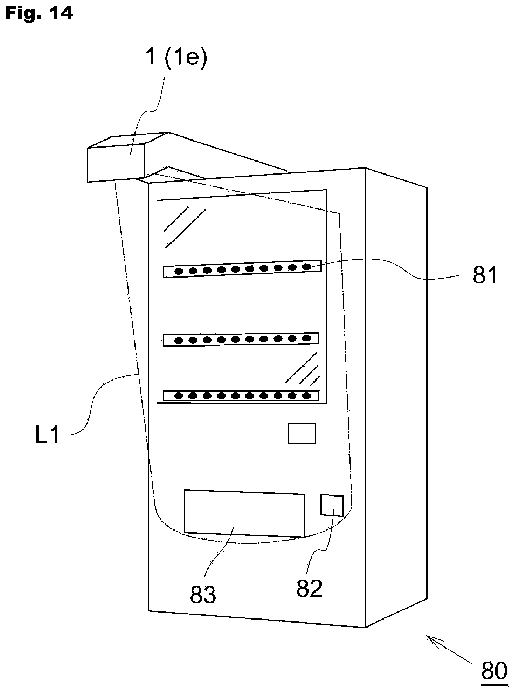

Fig. 14 shows a situation in which the inactivating apparatus 1 is installed for a vending machine 80. An inactivating apparatus 1e is installed for the purpose of performing inactivation of germs on, for example, an operating part 81, a coin return slot 82, and a dispensing slot 83 of the bending machine 80. Also in this example shown in Fig. 14, when the inactivating apparatus 1(1e) detects the absence of a human being (not shown), the integrated irradiation dose of ultraviolet light L1 per unit time is increased, and therefore the effect of inactivating germs on the operating part 81 and the slots (82, 83) is enhanced without allowing the integrated irradiation dose of ultraviolet light applied to a human being to exceed the standard value.

### Not according to the invention: J Z [Second Embodiment]

A second embodiment of the inactivating apparatus will be described by mainly focusing on different points from the first embodiment.

Compared with the first embodiment, the inactivating apparatus 1 of this embodiment differs only in the control content executed by the controller, and the rest is common.

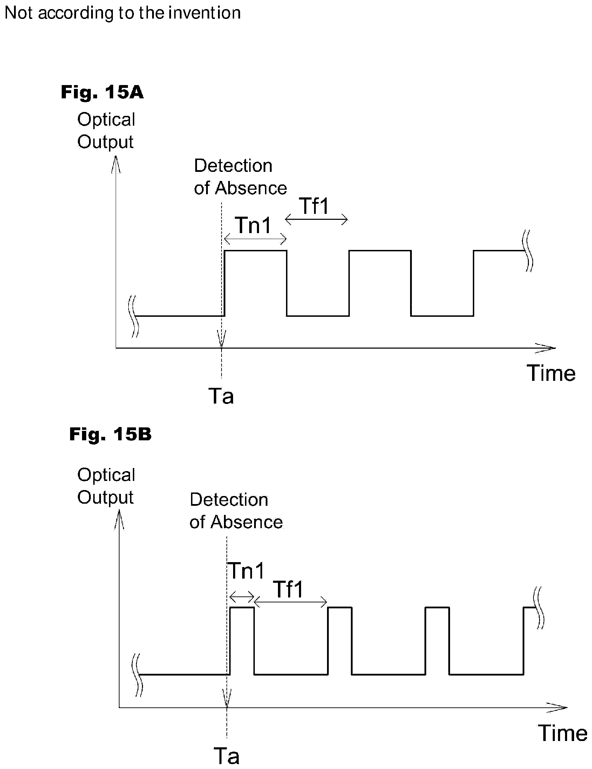

Fig. 15A is a timing chart schematically showing an example of the content of control executed by the controller 9 on the light source 2 in the inactivating apparatus 1 according to this embodiment. More specifically, Fig. 15A schematically shows a change in the optical output (emission intensity) of ultraviolet light L1 emitted from the light source 2 depending on the result of control executed by the controller 9.

In this embodiment, the controller 9 controls the light source 2 so that when the absence of a human being in the detection target area 40 is detected by the detector 20, a time when the light source 2 is energized (ON time) and a time when the light source 2 is not energized (OFF time) occur alternately. In other words, the controller 9 is configured to allow the light source 2 to execute an operation mode in which a turn-on operation and a turn-off operation are repeated (corresponding to a "first specific operation mode") in a time zone during which the absence of a human in the detection target area 40 is detected. In this case, the turn-on operation corresponds to "first control" and the turn-off operation corresponds to "second control". When explained using these terms, the specific operation mode is an operation mode to repeat an operation in which the first control is executed for a certain time and then the second control is executed for a certain time. Note that the "first operation control mode" may correspond to the "specific operation mode" in the first embodiment.

When the presence of a human being in the detection target area 40 is detected by the detector 20, the inactivating apparatus 1 according to this embodiment allows the controller 9 to control the light source 2 to turn off. This operation control mode corresponds to a "second operation control mode". Then, when the detector 20 again detects the absence of a human being in the detection target area 40, the operation control mode executed by the controller 9 is shifted from the second operation control mode to the first operation control mode.

For example, when the detector 20 detects the absence of a human being at a time Ta, the light source 2 is then energized for a time Tn1 so that the light source 2 emits ultraviolet light L1 for the time Tn1. After that, the energization to the light source 2 is stopped for a time Tf1 so that irradiation with ultraviolet light L1 is stopped. In the case shown in Fig. 15A, the time Tn1 and the time Tf1 are set to be the same and are both 5 minutes. In this case, the ratio of the continuous lighting time of the light source 2 (ON duty ratio) to the time required from once the light source 2 is turned on to the next time is 50%. The time Tn1 and the time Tf1 can appropriately be adjusted, for example, be set within 10 seconds to 10 minutes.

Fig. 15B is another timing chart schematically showing an example of the content of control executed by the controller 9 on the light source 2. As shown in Fig. 15B, the ON time (lighting time) Tn1 may be set to be shorter than the OFF time (light-off time) Tf1. For example, the time Tn1 is 15 seconds, and the time Tf1 is 1 minute. In this case, the ratio of the continuous lighting time of the light source 2 (ON duty ratio) to the time required from once the light source 2 is turned on to the next time is 20%. Thus, in this embodiment, in a time zone during which a human being is absent in the detection target area 40, ON/OFF control for the light source 2 is executed by the controller 9 within the range where the ON duty ratio is 50% or less.

Therefore, also in a time zone during which the absence of a human being is detected, the amount of electric power fed to the light source 2 is suppressed by the execution of ON/OFF control to the light source 2. As a result, the amount of heat generated by the power supply circuit for supplying electric power to the light source 2 is suppressed, which eliminates the need to provide a large cooling device in the inactivating apparatus 1.

The light source 2 equipped with the inactivating apparatus 1 is configured to emit ultraviolet light L1 having an optical output in a specific wavelength band belonging to a range of 190 nm to 235 nm. In the case of ultraviolet light L1 in such a wavelength band, a higher inactivating effect can be achieved without continuous irradiation of ultraviolet light compared to ultraviolet light with a wavelength of 254 nm emitted from a low-pressure mercury lamp or the like generally widely used for sterilization or the like. That is, as shown in the timing charts of Figs. 15A and 15B, a high inactivating effect can be achieved even if there is a time zone during which irradiation with ultraviolet light L1 from the light source 2 is not performed (time Tf1) in a time zone during which the absence of a human being is detected.

As described above, the inactivating apparatus 1 according to this embodiment controls the light source 2 to turn on intermittently in a time zone duringwhich the absence of a human being in the detection target area 40 is detected by the detector 20. This means that there is a time zone during which irradiation with ultraviolet light L1 is not performed (time Tf1). However, since the ultraviolet light L1 has an optical output in a specific wavelength band belonging to a range of 190 nm to 235 nm, the occurrence of photoreactivation of germs is inhibited in this time zone during which irradiation with ultraviolet light L1 is not performed. As a result, a high inactivating effect on germs is achieved.

The same is true for a case where the light source 2 is off in a time zone during which a human being is present. That is, even when irradiation with ultraviolet light L1 is stopped in a time zone during which a human being is present, irradiation with ultraviolet light L1 having an optical output in a specific wavelength band belonging to a range of 190 nm to 235 nm has been performed in a time zone during which a human being is present before that, and therefore the photoreactivation function of germs is inhibited.

Further, it is not necessary to continuously perform irradiation with ultraviolet light L1 at a high irradiance in a time zone during which the absence of a human being is detected, and therefore the amount of heat generated in the power supply circuit for the light source 2 is suppressed. In other words, the inactivating apparatus 1 makes it possible to suppress the temperature rise of the power supply circuit while ensuring the inactivation effect of germs.

The frequency of a change from the presence of a human being to the absence of a human being in the detection target area 40, or conversely, from the absence of a human being to the presence of a human being, depends on the installation location or usage of the inactivating apparatus 1. Even in the case where the inactivating apparatus 1 is installed in the room 50 such as a conference room, as shown in Fig. 1, a situation may occur where all the participants have left the room 50 after the conference, and one of the participants returns to the room 50 to pick up his/her belongings, and then leaves the room 50 again soon after picking up the belongings.

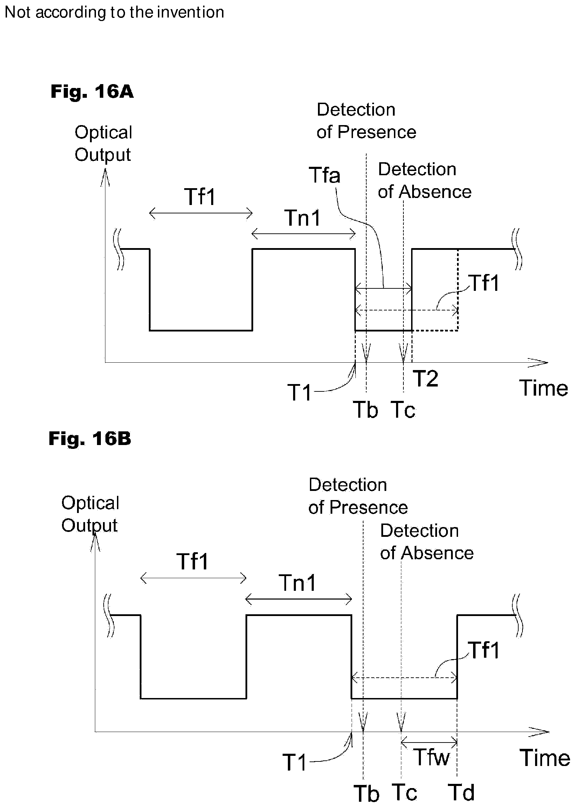

Fig. 16A is a timing chart of a case where the detector 20 has detected the absence of a human being in the detection target area 40 in a time zone before a time Tb, and the controller 9 has executed an operation control mode (first operation control mode) in which a turn-on operation and a turn-off operation are repeated on the light source 2. Assume that during this control, the detector 20 detects the presence of a human being in the detection target area 40 at a time Tb not long after the light source 2 is shifted from an on-state (a lit state) to an off-state (an unlit state) at a time T1 before the time Tb.

As described above, when the detector 20 detects the presence of a human being, the controller 9 shifts the operation control mode from the first operation control mode to the second operation control mode. The time Tb is in a time zone during which turn-off control is executed on the light source 2 even during the first operation control mode, and therefore the light source 2 is still kept turned off.

Assume that the detector 20 detects the absence of a human being at a time Tc after a lapse of a short time from the time Tb. This situation is presumed to occur when, as described above as an example, a human being enters the room 50 to pick up his/her belongings at the time Tb and then leaves the room 50 at the time Tc.

In the above situation, consider the case where the controller 9 immediately shifts the operation control mode from the second operation control mode to the first operation control mode and executes turn-on control on the light source 2 because the detector 20 has detected the absence of a human being at the time Tc. In this case, as shown in Fig. 16A, turn-on control is executed on the light source 2 at substantially the same time as the time Tc or at a time T2 after a lapse of a very short time from the time Tc. Then, a continuous light-off time immediately before the time T2 becomes Tfa, which is at risk of becoming much shorter than the continuous light-off time (Tf1) that is set when the first operation control mode is normally executed. As a result, the ON duty ratio of the light source 2 temporarily increases.

The frequency with which a human being enters and leaves the detection target area 40 cannot be controlled by the inactivating apparatus 1. Therefore, the frequency of lighting control may increase as shown in Fig. 16A, and in this case, the ON duty ratio of the light source 2 may increase, which may cause the heat generation of the power supply circuit to increase.

From such a viewpoint, as shown in Fig. 16B, it is preferred that the operation control mode of the controller 9 is shifted from the second operation control mode to the first operation control mode after a lapse of a transition waiting period Tfw from the time Tc at which the detector 20 has detected the absence of a human being. According to this configuration, even if the time interval between the time Tb when the presence of a human being is detected immediately before and the time Tc when the absence of a human being is detected again is short, an increase in ON duty ratio can be suppressed because a continuous light-off time is set longer.

Note that the value of the transition waiting period Tfw may be set by calculating backward from a time Td when the time Tf1 or a time longer than Tf1, Tf1 being preset in the first operation control mode, has elapsed since the light source 2 was turned off immediately before.

The situation described referring to Figs. 16A and 16B corresponds to the case where the presence/absence of a human being in the detection target area 40 changes in the time when the light source 2 is turned off during the execution of the first operation control mode. On the other hand, a similar phenomenon may occur also in the case where the presence/absence of a human being in the detection target area 40 changes in the time when the light source 2 is turned on during the execution of the first operation control mode.

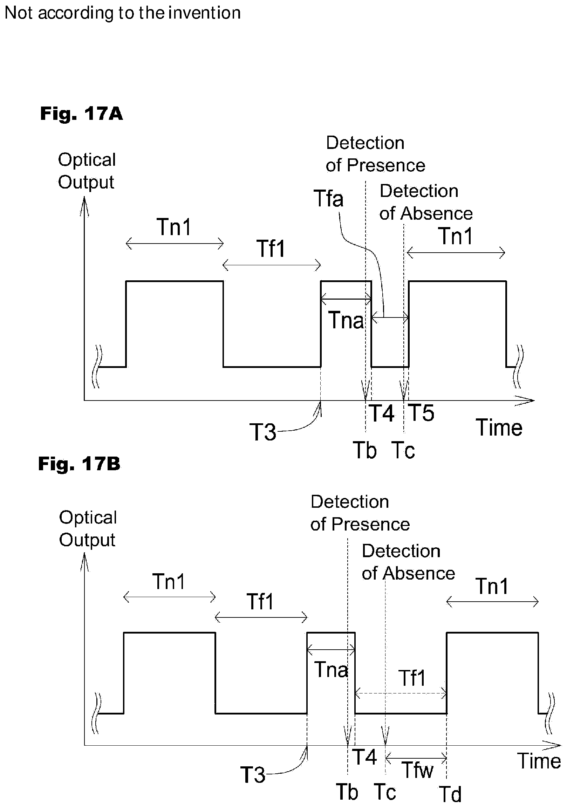

Similarly to Fig. 16A, Fig. 17A is a timing chart of a case where the detector 20 has detected the absence of a human being in the detection target area 40 in a time zone before a time Tb, and the controller 9 has executed an operation control mode (first operation control mode) in which a turn-on operation and a turn-off operation are repeated on the light source 2. Assume that during this control, the detector 20 detects the presence of a human being in the detection target area 40 at a time Tb not long after the light source 2 is shifted from an off-state (an unlit state) to an on-state (a lit state) at a time T3 before the time Tb.

As described above, when the detector 20 detects the presence of a human being, the controller 9 shifts the operation control mode from the first operation control mode to the second operation control mode. The time Tb is in a time zone during which turn-on control is executed on the light source 2, and therefore the controller 9 execute turn-off control on the light source 2 at substantially the same time as the time Tb or at a time T4 after a lapse of a very short time from the time Tb.

Assume that the detector 20 detects the absence of a human being at a time Tc after a lapse of a short time from the time Tb. Similarly to the case shown in Fig. 16A, consider the case where the controller 9 immediately shifts the operation control mode from the second operation control mode to the first operation control mode and executes turn-on control on the light source 2 because the detector 20 has detected the absence of a human being at the time Tc. In this case, as shown in Fig. 17A, turn-on control is executed on the light source 2 at substantially the same time as the time Tc or at a time T5 after a lapse of a very short time from the time Tc. Then, a continuous light-off time immediately before the time T5 becomes Tfa, which is at risk of becoming much shorter than the continuous light-off time (Tf1) that is set when the first operation control mode is normally executed.

Even if a continuous lighting time Tna between the time T3 and the time T4 is short, a continuous lighting time after the time T5 becomes Tn1, which is set during the execution of the first operation control mode. As a result, the ON duty ratio of the light source 2 temporarily increases before and after a time zone between the time T4 and the time T5. This may cause the heat generation of the power supply circuit to increase for the same reason as the situation described referring to Figure 16a.

From such a viewpoint, as shown in Fig. 17B, it is preferred that the operation control mode of the controller 9 is shifted from the second operation control mode to the first operation control mode after a lapse of a transition waiting period Tfw from the time Tc at which the detector 20 has detected the absence of a human being. According to this configuration, even if the time interval between the time Tb when the presence of a human being is detected immediately before and the time Tc when the absence of a human being is detected again is short, an increase in ON duty ratio can be suppressed because a long continuous light-off time is set longer. Even in the case shown in Fig. 1 7B, similarly to the case described above with referring to Fig. 16B, the value of the transition waiting period Tfw may be set by calculating backward from a time Td when the time Tf1 or a time longer than Tf1, Tf1 being preset in the first operation control mode, has elapsed since the light source 2 was turned off immediately before.

### [Other Embodiments]

Hereinbelow, other embodiments will be described.

<1 > The above embodiments have been described in the case where the inactivating apparatus 1 includes a human detecting sensor as the detector 20 for detecting the presence/absence of a human being. However, if a detecting means such as a separate human detecting sensor is previously provided in a place where the inactivating apparatus 1 is to be installed, the inactivating apparatus 1 may be configured to include a receiver that receives a signal from the detecting means as the detector 20.

Furthermore, the detector 20 is not particularly limited as long as the presence/absence of a human being can directly or indirectly be determined. For example, the detector 20 may judge the presence/absence of humans by using contact sensors, weight sensors, door sensors, etc. alone or in combination, or it may judge that based on the turning on/off of lighting fixtures installed in the space where germs are to be inactivated, or the opening/closing of room locks.

Lighting the light source 2 continuously may cause the power supply circuit (inverter, etc.) installed to supply power to the light source 2 to generate heat. Additionally, if the detector 20 malfunctions, there is a risk that ultraviolet light L1 will continue to be irradiated from the light source 2 even though a human being is present. From such a viewpoint, it is preferred that the controller 9 is previously set so that irradiation with ultraviolet light L1 is intermittently performed even when a human being is absent.

Not according to the invention:
<3> As shown in Fig. 19, the controller 9 may control the light source 2 to turn on continuously at a low output in the time zone during which humans are present in the detection area 40, while it may control the light source 2 to turn on periodically at a higher output after detection of the absence of a human being in the detection area 40 (time Ta).

Also in this case, similarly to the embodiments described above, the integrated irradiation dose of ultraviolet light L1 per unit time increases after the detection of the absence of a human being in the detection target area 40 and thus there is no concern that the integrated irradiation dose of ultraviolet light L1 irradiated to a human being will be increased. Therefore, the effect of inactivating germs in the room 50 (see Fig. 1) can be enhanced without exceeding the standard value specified by ACGIH or JIS Z 8812 for the integrated irradiation dose of ultraviolet light L1 irradiated to a human being.

On the other hand, when the room 50 is a conference room or the like, the time during which a human being continuously stays in the room 50 can be predicted to some extent, and it is unlikely that the human being will stay for a long time, such as 18 hours or more. Accordingly, even when a human being is present in the room 50, the effect of inactivation can be achieved without causing any impact on the human body by allowing the controller 9 to control the output of the light source 2 so that the ultraviolet light L1 is irradiated at an extremely low irradiance within the range that does not exceed the standard value specified by ACGIH and JIS Z 8812.

In this case, the controller 9 preferably controls so that the longer the time from the detection of the presence of a human being in the detection target area 40 immediately before until the detection of the absence of a human being in the detection target area 40, in other words, the longer the continuous presence of a human being in the detection target area 40, the higher the integrated irradiation dose of ultraviolet light L1 per unit time. More specifically, as the continuous presence time of a human being in the detection target area 40 increases, the optical output of the light source 2 in a time zone during which a human being is absent just after that or the ON duty ratio may be set to be higher.

<4> The above embodiments have been described regarding a case where excimer lamps 3 are provided as a light source 2. However, the structure of the light source 2 is not particularly limited as long as the light source 2 emits ultraviolet light L1 having an optical output in a specific wavelength band belonging to a range of 190 nm to 235 nm. For example, the light source 2 may be constituted from a solid light source such as an LED or a laser diode.

<5> The above embodiments have been described in the case where the "specific operation mode" corresponds to an operation mode in which the first control of executing a turn-on operation and the second control of executing a turn-off operation are repeated. However, the "specific operation mode" may correspond to an operation mode in which the first control of turning on the light source 2 at a relatively high emission intensity and the second control of turning on the light source 2 at an emission intensity relatively lower than that during the first control are repeated.

For example, the controller 9 adjusts the illuminance of the light source 2 so that the irradiance of ultraviolet light L1 on the light extraction face 10 during the execution of the first control is higher than 0.3 mW/cm² and that during the execution of the second control is less than 0.3 mW/cm². The classification value of the irradiance (irradiance standard value) may be arbitrarily set within a range of 0.1 mW/cm² to 3 mW/cm². For example, when the irradiance reference value of ultraviolet light L1 on the light extraction face 10 is set to a value selected from the group consisting of set values of 3 mW/cm², 2.5 mW/cm², 2 mW/cm², 1.5 mW/cm², 1 mW/cm², 0.5 mW/cm², and 0.1 mW/cm², the irradiance may be controlled to be higher than the irradiance reference value when the first control is executed and may be controlled to be less than the irradiance reference value when the second control is executed.

Furthermore, the irradiance of ultraviolet light L1 during the execution of the second control is preferably less than 50%, more preferably less than 30%, particularly preferably less than 15% of the irradiance of ultraviolet light L1 during the execution of the first control. When the irradiance of ultraviolet light L1 during the execution of the second control is less than 1% of the irradiance of ultraviolet light L1 during the execution of the first control, the second control may virtually be regarded as a "turn-off" operation.

<6> The above embodiments have been described in the case where the controller 9 controls the light source 2 depending on the detection result of the detector 20 after the detector 20 detects whether or not a human being is absent in the detection target area 40. However, the detector 20 may be configured to detect the absence of an animal other than a human being. In this case, the inactivating apparatus 1 according to the present invention includes a light source 2 that emits ultraviolet light L1 having an optical output in a specific wavelength band belonging to a range of 190 nm to 235 nm, a controller 9 that executes lighting control on the light source 2, and a detector 20 for detecting whether or not an "animal" is present in a detection target area 40, which is a part of an irradiation area of the ultraviolet light L1 or an area adjacent to the irradiation area. The controller may control the light source 2 to increase an integrated irradiation dose of the ultraviolet light L1 per unit time when the detector 20 detects the absence of an "animal".

In this case, the animal to be detected may be a pet such as a dog, a cat, or a rabbit, a domestic animal such as a cow, a pig, a chicken, or a horse, an animal reared in a zoo, or a protected animal.

### DESCRIPTION OF REFERENCE SIGNS

- 1 (1a, 1b, 1c, 1d, 1e): Inactivating apparatus
- 2: Light source
- 3: Excimer lamp
- 3G: Light-emitting gas
- 9: Controller
- 10: Light extraction face
- 12: Lamp house
- 12a: Main body casing
- 12b: Lid
- 18: Power feeder
- 20: Detector
- 40: Detection target area
- 50: Room
- 51: Desk
- 52: Chair
- 53: Wall paper
- 60: Aisle
- 61: Human being
- 62, 63: Area
- 70: Room
- 71: Operating part
- 80: Vending machine
- 81: Operating part
- 82: Slot
- 83: Slot
- L1: Ultraviolet light
- L2: infrared light

## Claims

1. An inactivating apparatus (1) for bacteria or viruses, comprising:
a light source (2) that emits ultraviolet light (L1) with an optical output in a specific wavelength band belonging to a range of 190 nm to 235 nm;
a controller (9) for lighting control of the light source (2); and
a detector (20) that detects whether or not a human being is present in a detection target area (40), which is a part of an irradiation area of the ultraviolet light (L1) or an area adjacent to the irradiation area,
wherein the controller (9) controls the light source (2) to increase an integrated irradiation dose of the ultraviolet light (L1) per unit time when the detector (20) detects the absence of a human being;
**characterised in that**
the controller (9) is configured to be capable of executing a specific operation mode in which a first control to turn on the light source (2) at a relatively high emission intensity and a second control to turn on the light source (2) at a relatively lower emission intensity than that during the execution of the first control or to turn off the light source (2) are repeated, and
the controller (9) continues to execute the specific operation mode with the integrated irradiation dose of the ultraviolet light (L1) per unit time increased when the detector (20) detects the absence of a human being during the execution of the specific operation mode.

2. The inactivating apparatus (1) for bacteria or viruses according to claim 1, wherein
the specific operation mode is a mode in which an operation of executing the first control for a first predetermined time and then executing the second control for a second predetermined time is repeated, and
the controller (9) executes at least one of increasing the first predetermined time or decreasing the second predetermined time when the detector (20) detects the absence of a human being.

3. The inactivating apparatus (1) for bacteria or viruses according to claim 1, wherein when the detector (20) detects the absence of a human being, the controller (9) controls to increase the optical output of the light source (2) during the execution of the first control.

4. The inactivating apparatus (1) for bacteria or viruses according to claim 1, wherein the ultraviolet light (L1) has a peak wavelength in a range of 200 nm to 230 nm.

## Patentansprüche

1. Inaktivierungsvorrichtung (1) für Bakterien oder Viren, umfassend:
eine Lichtquelle (2), die ultraviolettes Licht (L1) mit einer optischen Leistung in einem spezifischen Wellenlängenband emittiert, das zu einem Bereich von 190 nm bis 235 nm gehört,
ein Steuergerät (9) zur Beleuchtungssteuerung der Lichtquelle (2) und
einen Detektor (20), der erfasst, ob ein Mensch in einem Erfassungszielbereich (40), der ein Teil eines Bestrahlungsbereichs des ultravioletten Lichts (L1) oder ein an den Bestrahlungsbereich angrenzender Bereich ist, anwesend ist oder nicht,
wobei die Steuerung (9) die Lichtquelle (2) so steuert, dass eine integrierte Bestrahlungsdosis des ultravioletten Lichts (L1) pro Zeiteinheit erhöht wird, wenn der Detektor (20) die Abwesenheit eines Menschen erfasst,
**dadurch gekennzeichnet, dass**
die Steuerung (9) so konfiguriert ist, dass sie in der Lage ist, einen spezifischen Betriebsmodus auszuführen, in dem eine erste Steuerung zum Einschalten der Lichtquelle (2) mit einer relativ hohen Emissionsintensität und eine zweite Steuerung zum Einschalten der Lichtquelle (2) mit einer relativ niedrigeren Emissionsintensität als derjenigen während der Ausführung der ersten Steuerung oder zum Ausschalten der Lichtquelle (2) wiederholt werden, und
die Steuerung (9) die Ausführung des spezifischen Betriebsmodus mit der integrierten Bestrahlungsdosis des ultravioletten Lichts (L1) pro Zeiteinheit erhöht fortsetzt, wenn der Detektor (20) die Abwesenheit eines Menschen während der Ausführung des spezifischen Betriebsmodus feststellt.

2. Inaktivierungsvorrichtung (1) für Bakterien oder Viren nach Anspruch 1, wobei
der spezifische Betriebsmodus ein Modus ist, in dem ein Vorgang der Ausführung der ersten Steuerung eine erste vorbestimmte Zeit lang und dann der Ausführung der zweiten Steuerung eine zweite vorbestimmte Zeit lang wiederholt wird, und
die Steuerung (9) wenigstens eine Verlängerung der ersten vorbestimmten Zeit oder eine Verkürzung der zweiten vorbestimmten Zeit ausführt, wenn der Detektor (20) die Abwesenheit eines Menschen feststellt.

3. Inaktivierungsvorrichtung (1) für Bakterien oder Viren nach Anspruch 1, wobei, wenn der Detektor (20) die Abwesenheit eines Menschen feststellt, die Steuerung (9) eine Erhöhung der optischen Leistung der Lichtquelle (2) während der Ausführung der ersten Steuerung steuert.

4. Inaktivierungsvorrichtung (1) für Bakterien oder Viren nach Anspruch 1, wobei das ultraviolette Licht (L1) eine Spitzenwellenlänge in einem Bereich von 200 nm bis 230 nm aufweist.

## Revendications

1. Appareil d'inactivation (1) pour bactéries ou virus, comprenant :
une source de lumière (2) qui émet de la lumière ultraviolette (L1) avec une sortie optique dans une bande de longueur d'onde spécifique appartenant à une plage de 190 nm à 235 nm ;
un contrôleur (9) pour commander l'éclairage de la source de lumière (2) ; et
un détecteur (20) qui détecte si oui ou non un être humain est présent dans une zone cible de détection (40), qui est une partie d'une zone d'irradiation de la lumière ultraviolette (L1) ou une zone adjacente à la zone d'irradiation,
dans lequel le contrôleur (9) commande la source de lumière (2) pour augmenter une dose d'irradiation intégrée de la lumière ultraviolette (L1) par unité de temps lorsque le détecteur (20) détecte l'absence d'un être humain ;
**caractérisé en ce que**
le contrôleur (9) est configuré pour être capable d'exécuter un mode d'opération spécifique dans lequel une première commande pour allumer la source de lumière (2) à une intensité d'émission relativement élevée et une seconde commande pour allumer la source de lumière (2) à une intensité d'émission relativement inférieure que celle durant l'exécution de la première commande ou éteindre la source de lumière (2) sont répétés et
le contrôleur (9) continue d'exécuter le mode d'opération spécifique avec la dose d'irradiation intégrée de la lumière ultraviolette (L1) par unité de temps augmentée lorsque le détecteur (20) détecte l'absence d'un être humain durant l'exécution du mode d'opération spécifique.

2. Appareil d'inactivation (1) pour bactéries ou virus selon la revendication 1, dans lequel
le mode d'opération spécifique est un mode dans lequel une opération d'exécution de la première commande durant un premier temps prédéterminé puis l'exécution de la seconde commande durant un second temps prédéterminé est répétée et
le contrôleur (9) exécute au moins un de l'augmentation du premier temps prédéterminé ou la diminution du second temps prédéterminé lorsque le détecteur (20) détecte l'absence d'un être humain.

3. Appareil d'inactivation (1) pour bactéries ou virus selon la revendication 1, dans lequel, lorsque le détecteur (20) détecte l'absence d'un être humain, le contrôleur (9) commande d'augmenter la sortie optique de la source de lumière (2) durant l'exécution de la première commande.

4. Appareil d'inactivation (1) pour bactéries ou virus selon la revendication 1, dans lequel la lumière ultraviolette (L1) a une longueur d'onde de pic dans une plage de 200 nm à 230 nm.
